(19) Europäisches Patentamt
European Patent Office
Office européen des brevets

(11) Numéro de publication: 0 329 520 B1

# (12) FASCICULE DE BREVET EUROPEEN

(45) Date de publication du fascicule du brevet: 23.05.90

(51) Int. Cl.⁵: **C07C 319/04,** C07C 321/04

(21) Numéro de dépôt: **89400328.4**

(22) Date de dépôt: **06.02.89**

(54) **Perfectionnement à la synthèse de mercaptans secondaires à partir d'oléfines linéaires.**

(30) Priorité: **17.02.88 FR 8801879**

(43) Date de publication de la demande: **23.08.89 Bulletin 89/34**

(45) Mention de la délivrance du brevet: **23.05.90 Bulletin 90/21**

(84) Etats contractants désignés: **AT BE CH DE ES FR GB GR IT LI LU NL SE**

(56) Documents cités: **FR-A- 2 531 426**

(73) Titulaire: **SOCIETE NATIONALE ELF AQUITAINE (PRODUCTION), Tour Elf 2, Place de la Coupole La Défense 6, F-92400 Courbevoie(FR)**

(72) Inventeur: **Arretz, Emmanuel, 2 Rue de Cagnes, F-64000 Pau(FR)**

(74) Mandataire: **Leboulenger, Jean et al, ATOCHEM Département Propriété Industrielle, F-92091 Paris la Défense 10 Cédex 42(FR)**

EP 0 329 520 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne le domaine des mercaptans et a plus particulièrement pour objet la fabrication de mercaptans secondaires à partir d'oléfines linéaires.

La préparation de mercaptans par action de l'hydrogène sulfuré sur une oléfine en présence d'un catalyseur constitué par une résine échangeuse de cations a été décrite dans le brevet FR 2 531 426 qui préconise de régler la température entre les limites rigoureuses de 45 et 75°C ou mieux entre 50 et 70°C. Ce procédé donne d'excellents résultats et est particulièrement performant pour l'obtention de mercaptans tertiaires comme le tertiobutylmercaptan, le tertiononylmercaptan et le tertiododécylmercaptan à partir respectivement de l'isobutylène, du propylène trimère et du propylène tétramère dont les taux de conversion sont très élevés (90-98 %) dans le domaine de températures préconisé.

Il n'en est malheureusement pas de même lorsqu'on applique le procédé du brevet précité aux oléfines linéaires, qu'il s'agisse d'α-oléfines ou d'oléfines internes, pour obtenir les mercaptans secondaires de formule générale :

$$R_1 - \underset{SH}{\underset{|}{CH}} - R_2$$

où $R_1$ et $R_2$ sont des groupes hydrocarbonés linéaires, tels que la chaîne $R_1$ - CH - $R_2$ contienne de 3 à 30 atomes de carbone. Il a en effet été constaté que les oléfines linéaires, en particulier les oléfines linéaires à bas poids moléculaire, ont une réactivité nettement moins importante que celle des oléfines tertiaires, vis-à-vis de $H_2S$ sur des résines catalytiques, comme l'Amberlyst 15, dans l'intervalle de température de 45 à 75°C, et ce d'autant plus si l'on opère en phase gazeuse. Ainsi par exemple à 60°C, les taux de conversion du butène-1, de l'hexène-1 et du dodécène-1 sont de l'ordre de 35 à 45 % seulement.

Il a maintenant été trouvé que la réactivité des oléfines linéaires peut être très fortement accrue en travaillant à une température supérieure à 75°C et de préférence comprise entre 90 et 120°C. Ce résultat n'était pas prévisible car, pour d'autres oléfines, le taux de conversion est pratiquement constant entre 0 et 100°C (diisobutylène par exemple) ou décroît lorsque la température augmente (triisobutylène par exemple).

Le procédé selon l'invention pour la préparations de mercaptans secondaires par réaction de l'hydrogène sulfuré avec une oléfine linéaire est donc caractérisé en ce que l'on effectue la réaction à une température supérieure à 75°C, de préférence entre 90 et 120°C, en présence d'un catalyseur constitué par une résine échangeuse de cations.

Comme catalyseur à utiliser selon l'invention conviennent tous les différents polymères et copolymères à fonctions acides, connus dans l'art comme échangeurs de cations. En particulier, on peut employer des résines à base de polystyrène sulfoné réticulées, en particulier avec du divinylbenzène, des résines acryliques ou phénylacryliques à groupes carboxyliques libres, des résines du type phénol-formaldéhyde dérivées des acides phénol-sulfoniques, des échangeurs ligno-sulfoniques, etc... Des résines de ce genre se trouvent dans le commerce sous différentes dénominations, en particulier Allassion, Cecacit, Wofatites, Levatites, Imac, Ionac, Amberlites, Liquorex, Zeorex, Zeocarb, Dowex, etc... Conviennent tout particulièrement les copolymères sulfonés du styrène avec le divinyl benzène, par exemple ceux que l'on trouve dans le commerce sous les dénominations Amberlyst, Lewatit ou Dowex ; d'autre part, peuvent être employés avantageusement les copolymères de tétrafluoroéthylène avec un acide perfluorosulfonique (en particulier l'acide perfluoro-3,6-dioxa-4-méthyl-7-octène sulfonique) connus sous la marque Nafion. Quelle que soit la résine employée comme catalyseur, il faut veiller à ce qu'elle ne contienne pas plus de 0,5 % d'eau déterminable après 6 heures de séchage à 80°C et soit de préférence aussi sèche que possible (avantageusement moins de 0,2 % d'eau).

Bien qu'il soit possible d'opérer à toute pression relative entre 1 et 50 bars, on obtient d'excellents résultats en travaillant sous des pressions modérées, notamment de 10 à 16 bars, cette zone étant industriellement la plus favorable pour travailler avec $H_2S$ gazeux.

Comme dans les procédés connus, il convient d'employer un certain excès d'hydrogène sulfuré, les rapports molaires pratiques $H_2S$/oléfine linéaire étant de 1,2 à 10 et, de préférence, de 3 à 8.

Le procédé selon l'invention peut s'appliquer à toute oléfine linéaire ayant jusqu'à 30 atomes de carbone. Il présente cependant un intérêt tout particulier pour la fabrication des mercaptans secondaires contenant de 3 à 16 atomes de carbone qui sont utilisés comme réactifs de synthèse de disulfures et de certains agents tensio-actifs.

Le procédé selon l'invention peut être mis en oeuvre en discontinu ou en continu suivant toute méthode connue en soi. On préfère cependant opérer en continu, dans un réacteur agité ou dans un réacteur tubulaire, chargé de catalyseur et alimenté de manière continue en hydrogène sulfuré et en oléfine linéaire. Dans certains cas, il peut être avantageux de recycler au réacteur la fraction d'oléfine non transformée.

Les exemples suivants illustrent l'invention, sans la limiter.

EXEMPLE 1 : SYNTHESE DU BUTANETHIOL-2

Dans un réacteur tubulaire d'une capacité d'environ 420 ml (longueur : 135 cm ; diamètre intérieur : 20 mm) on place 200 ml de résine sulfonée Amberlyst 15 sèche. Sous une pression de 15 bars on introduit en tête du réacteur, en continu, du butène-1 liquide à raison de 56 g/h (soit 1 mole/h) et 136 g/h de $H_2S$ gazeux (soit 4 moles par mole de butène-1). Ces réactifs sont mélangés intimement avant leur passage dans la réacteur où le mélange réactionnel est maintenu à la température de 75°C. Le liquide s'écoulant en continu du réacteur est recueilli et est analysé ainsi que les effluents gazeux éliminés du réacteur. Cet essai donne une conversion du butène-1 en composés soufrés de 53 %.

En maintenant les mêmes débits de réactifs et la même pression opératoire, on a effectué trois autres essais en opérant à 90°C, 100°C et 120°C respectivement. Les résultats de ces essais sont présentés dans le tableau suivant :

| Temperature (°C) | Conversion Butène-1 | Sélectivité* Butanethiol-2 | Sélectivité* Dibutylsulfure |
|---|---|---|---|
| 75 | 53 | 86 | 12 |
| 90 | 65 | 80 | 18 |
| 100 | 72 | 74 | 23 |
| 120 | 85 | 70 | 28 |

* Sélectivités calculées par rapport au butène transformé

EXEMPLE 2 : SYNTHESE DE L'HEXANETHIOL-2

On a travaillé comme à l'exemple 1, mais en remplaçant le butène-1 par de l'hexène-1 liquide injecté à raison de 84 g/h. Quatre essais ont été effectués à 60, 90, 100 et 120°C respectivement et leurs résultats déterminés par analyse des effluents liquides et gazeux, sont présentés dans le tableau suivant :

| Temperature (°C) | Conversion Hexène-1 | Sélectivité Hexanethiol-2 | Sélectivité Dihexylsulfure |
|---|---|---|---|
| 60 | 45 | 93 | 3 |
| 90 | 71 | 84 | 7 |
| 100 | 83 | 83 | 9 |
| 120 | 88 | 81 | 12 |

EXEMPLE 3 : SYNTHESE DE DODECANETHIOL-2

On a répété l'exemple 1, mais en remplaçant le butène-1 par du dodécène-1 liquide injecté à raison de 168 g/h et en travaillant à 60, 100 et 120°C. L'analyse des effluents de sortie du réacteur donne les résultats présentés dans le tableau suivant :

| Temperature (°C) | Conversion Dodecène-1 | Sélectivité Dodecanethiol-2 | Sélectivité Didedecylsulfure |
|---|---|---|---|
| 60 | 47 | 92 | 4 |
| 100 | 85 | 81 | 11 |
| 120 | 89 | 79 | 14 |

## Revendications

1. Procédé de préparation de mercaptans secondaires à partir d'oléfines linéaires et d'hydrogène sulfuré par catalyse hétérogène, caractérisé en ce que l'on effectue la réaction à une température supérieure à 75°C en présence d'un catalyseur constitué par une résine échangeuse de cations.

2. Procédé selon la revendication 1, dans lequel on opère à une température comprise entre 90 et 120°C.

3. Procédé selon la revendication 1 ou 2, dans lequel la résine échangeuse de cations est un copolymère sulfoné styrène-divinylbenzène ou un copolymère tétrafluoroéthylène-acide perfluorosulfonique.

4. Procédé selon l'une des revendications 1 à 3, dans lequel on opère sous une pression comprise entre 1 et 50 bars, de préférence entre 10 et 16 bars.

5. Procédé selon l'une des revendications 1 à 4, dans lequel le rapport molaire $H_2S$/oléfine linéaire est compris entre 1,2 et 10, de préférence entre 3 et 8.

6. Procédé selon l'une des revendications 1 à 5, dans lequel l'oléfine linéaire contient de 3 à 30 atomes de carbone, et de préférence, 3 à 16.

7. Procédé selon l'une des revendications 1 à 6, dans lequel on recycle l'oléfine linéaire non transformée.

## Claims

1. Process for the preparation of secondary mercaptans from linear olefins and from hydrogen sulphide by means of heterogeneous catalysis, characterized in that the reaction is carried out at a temperature above 75°C in the presence of a catalyst consisting of a cation exchange resin.

2. Process according to Claim 1, in which the operation is carried out at a temperature of between 90 and 120°C.

3. Process according to Claim 1 or 2, in which the cation exchange resin is a sulphonated styrene-divinylbenzene copolymer or a tetrafluoroethylene-perfluorosulphonic acid copolymer.

4. Process according to one of Claims 1 to 3, in which the operation is carried out at a pressure of between 1 and 50 bars, preferably between 10 and 16 bars.

5. Process according to one of Claims 1 to 4, in which the molar ratio $H_2S$/linear olefin is between 1.2 and 10, preferably between 3 and 8.

6. Process according to one of Claims 1 to 5, in which the linear olefin contains from 3 to 30, and preferably 3 to 16, carbon atoms.

7. Process according to one of Claims 1 to 6, in which the unconverted linear olefin is recycled.

## Revendications

1. Verfahren zur Herstellung sekundärer Mercaptane ausgehend von linearen Olefinen und Schwefelwasserstoff durch heterogene Katalyse, dadurch gekennzeichnet, daß man die Reaktion bei einer Temperatur oberhalb von 75°C in Gegenwart eines Katalysators bestehend aus einem Kationenaustauscherharz durchführt.

2. Verfahren nach Anspruch 1, bei dem man bei einer Temperatur zwischen 90 und 120° C arbeitet.

3. Verfahren nach Anspruch 1 oder 2, bei dem das Kationenaustauscherharz ein sulfoniertes Styrol-Divinylbenzol-Copolymer oder ein Tetrafluorethylen-Perfluorsulfonsäure-Copolymer ist.

4. Verfahren nach einem der Ansprüche 1 bis 3, bei dem man bei einem Druck zwischen 1 und 50 bar, vorzugsweise zwischen 10 und 16 bar arbeitet.

5. Verfahren nach einem der Ansprüche 1 bis 4, bei dem das molare Verhältnis $H_2S$/lineares Olefin zwischen 1,2 und 10, vorzugsweise zwischen 3 und 8 beträgt.

6. Verfahren nach einem der Ansprüche 1 bis 5, bei dem das lineare Olefin 3 bis 30 Kohlenstoffatome, vorzugsweise 3 bis 16 Kohlenstoffatome enthält.

7. Verfahren nach einem der Ansprüche 1 bis 6, bei dem man das nicht umgewandelte lineare Olefin zurückführt.